# FASCICULE DE BREVET EUROPEEN

(11) **EP 3 148 402 B1**
(45) Date de publication et mention de la délivrance du brevet: **13.01.2021**
(21) Numéro de dépôt: 15739308.3
(22) Date de dépôt: 27.05.2015
(51) Int. Cl.: A61B 1/00, A61B 1/24, G02B 27/01, G06T 19/00, A61C 9/00

(54) **DISPOSITIF DE VISUALISATION DE L'INTERIEUR D'UNE BOUCHE D'UN PATIENT**
VORRICHTUNG ZUR BETRACHTUNG DER INNENSEITE DES MUNDES EINES PATIENTEN
DEVICE FOR VIEWING THE INSIDE OF THE MOUTH OF A PATIENT

(30) Priorité: 27.05.2014 FR 1454774; 27.05.2014 FR 1461539
(43) Date de publication de la demande: 05.04.2017
(73) Titulaire: Duret, François, 11560 Fleury d'Aude (FR); Querbes, Olivier, 31750 Esqualquens (FR); Querbes-Duret, Véronique, 31750 Esqualquens (FR); Condor SAS, 11560 Fleury d'Aude (FR)
(72) Inventeur: DURET, François, 11560 Fleury D'aude (FR); QUERBES, Olivier, 31750 Esqualquens (FR); QUERBES-DURET, Véronique, 31750 Esqualquens (FR)
(74) Mandataire: Rhein, Alain
(86) Numéro de dépôt international: PCT/FR2015/000104
(87) Numéro de publication internationale: WO 2015/181454

(56) Documents cités:
- WO-A1-2008/149172
- WO-A1-2014/047402
- WO-A2-2004/100067
- US-A1- 2012 056 993
- US-A1- 2012 289 777

## Description

La présente invention se rapporte à un dispositif de visualisation de l'intérieur d'une bouche d'un patient. Elle se rapporte également à un dispositif de mesure endobuccale.

On connait un dispositif de visualisation de l'intérieur d'une bouche d'un patient comprenant une caméra endobuccale adaptée à prendre une empreinte optique de la bouche d'un patient et un écran de visualisation déporté permettant à un utilisateur du dispositif de visualiser les images prises par la caméra endobuccale. Un tel dispositif est notamment utilisé pour la réalisation de prothèses dentaires. Le document US 2012/056993 décrit, par exemple un tel dispositif de visualisation selon la préambule de la revendication 1.

L'inconvénient d'un tel dispositif est que l'utilisateur doit constamment regarder l'écran déporté où s'affichent des images correspondantes à celles prises par la caméra endobuccale (soit les images prises par la caméra, soit des images issues d'un traitement informatique de modélisation à partir des images prises par la caméra), l'obligeant ainsi à détourner son regard de la bouche du patient et l'empêchant d'avoir une correspondance entre la bouche du patient où il effectue son travail et ce qui est affiché à l'écran (par exemple, le résultat réel de son travail, ou la représentation de ce que devrait être au final son travail) .

Ainsi, lors d'une prise d'empreinte optique, l'utilisateur doit balayer manuellement avec la caméra endobuccale l'intégralité de la zone de la bouche concernée par la prise d'empreinte. Il doit donc suivre l'évolution de la prise d'empreinte qui peut être affichée sur l'écran déporté, et, par conséquent, quitter du regard la bouche du patient.

Le déplacement du regard peut entrainer des mouvements néfastes, imprécis et incontrôlés des mains de l'utilisateur qui peuvent provoquer une blessure au patient (dans la bouche ou à l'extérieur de celle-ci). Cet inconvénient peut être plus important si l'utilisateur prend une empreinte optique dynamique en trois dimensions.

En outre, le déplacement du regard est également très fatigant, d'autant plus quand le rythme de ce déplacement est très élevé, par exemple plus de vingt mouvements des yeux aller et retour par minute.

Dans le cas où le dispositif de visualisation utilise un projecteur de lumière active structurée, la corrélation des images réelles modélisées avec des informations issues de la réalité augmentée est très difficile du fait de la projection de masques (point, ligne, grille) sur les dents ou la gencive.

De plus, si des indications supplémentaires aux images prises par la caméra endobuccales sont également affichées à l'écran, l'utilisateur doit alors faire l'effort supplémentaire de transposer mentalement ces indications jusqu'au champ opératoire, avec un risque accru d'être imprécis ou de mal faire son travail.

Malgré l'existence de dispositifs à réalité augmentée, il n'est pas possible de les utiliser, la corrélation ne pouvant être réalisée entre la vue réelle de la bouche du patient et l'information issue de la réalité augmentée. Même si l'information issue de la réalité augmentée apparaissait sur l'écran déporté, la visualisation de l'écran déporté par l'utilisateur ne serait jamais effectuée en temps réel et son geste ne serait pas positionné précisément dans son champ de travail.

L'écran déporté a également une influence sur la qualité de l'empreinte optique : l'utilisateur n'a jamais de corrélation réelle et directe lui permettant de savoir si la prise d'empreinte est complète ou non. Il risque également de bouger la caméra endobuccale de manière incontrôlée à chaque mouvement oculaire, ce qui rend les résultats obtenus incertains.

La visualisation sur un écran déporté de la représentation modélisée des organes de la bouche (par exemple, la représentation de ce que devraient être les dents et la gencive à la fin du travail, une fois la prothèse dentaire posée) ne peut être réalisée en même temps que la visualisation en direct des mêmes organes. Il en est de même pour toutes les informations issues de logiciels relatifs à la réalité augmentée affichées sur l'écran déporté.

Le regard de l'utilisateur ne voit pas forcément toutes les zones accessibles à la caméra endobuccale, ce qui rend difficile, voire impossible, l'estimation de ce qui a été mesuré. Cet inconvénient est d'autant plus vrai si la caméra endobuccale est un dispositif du type porte-empreinte optique qui est volumineux et cache une partie importante de la bouche du patient.

Du fait que l'utilisateur ne peut pas voir la bouche du patient et le résultat de son travail confondus en temps réel dans un même référentiel, son geste ne peut être sécurisé.

La présente invention vise à pallier les inconvénients précités.

L'invention concerne un dispositif de visualisation de l'intérieur d'une bouche d'un patient, le dispositif comprenant une caméra adaptée à prendre une empreinte optique d'organes disposés dans la bouche, caractérisé en ce qu'il comprend une paire de lunettes à réalité augmentée ayant, d'une part, un verre optique au travers duquel un utilisateur de la paire de lunettes peut voir l'intérieur de la bouche, et, d'autre part, une caméra de visualisation filmant ce que l'utilisateur voit au travers du verre optique, des premières images correspondantes à celles prises par la caméra de visualisation étant corrélées avec des secondes images correspondantes à celles prises par la caméra de prise d'empreinte optique, les secondes images pouvant être projetées sur le verre optique.

Ainsi, l'invention propose un dispositif de visualisation qui permet de réunir dans un même champ de vision (le verre optique, et donc la bouche du patient), de façon corrélée, la visualisation directe de l'intérieur de la bouche du patient et les secondes images qui correspondent à celles prises par la caméra de prise d'empreinte optique. Ces secondes images peuvent être aussi bien les images prises par la caméra de prise d'empreinte optique que des images issues d'une modélisation informatique des images prises par cette caméra.

Si l'utilisateur le souhaite, il peut donc suivre l'évolution de son travail (par exemple, l'établissement d'un diagnostic ou la réalisation d'une empreinte) sans quitter des yeux la bouche du patient.

Par la présence d'un unique champ de vision, l'utilisateur ne risque plus de faire des mouvements néfastes, imprécis et incontrôlés de ses mains, avantage d'autant plus important s'il travaille en mesure dynamique en trois dimensions.

Par la suppression du détournement de son regard hors de la bouche du patient, l'utilisateur ne risque plus de provoquer de blessure au patient. Les gestes de l'utilisateur et les informations l'aidant pour la réalisation de son travail sont en permanence dans un même champ de vision.

Du fait de l'absence de mouvement des yeux (notamment à un rythme très élevé), l'utilisateur est nettement moins fatigué par l'usage du dispositif conforme à la présente invention.

La corrélation entre la vue réelle et la vue modélisée permet à l'utilisateur de pouvoir utiliser tout type de méthode de prise d'empreinte optique, que ce soit ou non une méthode utilisant une lumière active structurée.

Le dispositif selon l'invention permet d'avoir une restitution naturelle stéréoscopique en trois dimensions sans être obligé d'utiliser des écrans à trois dimensions qui sont toujours chers et souvent peu performant.

La caméra de prise d'empreinte optique est, par définition, une caméra qui permet de prendre une empreinte sans contact.

La caméra de prise d'empreinte optique peut être une caméra portée par la paire de lunettes à réalité augmentée, une caméra endobuccale (déplaçable manuellement), ou un porte-empreinte optique La caméra de prise d'empreinte optique peut être un système de porte-empreinte optique endobuccal (aussi bien un système de porte-empreinte optique relativement encombrant qui comporte des dispositifs de projection et de lecture et qui utilise, soit l'interférométrie holographique, soit des fibres optiques conduisant des images moirés, qu'un système de porte-empreinte optique qui utilise le principe de la stéréoscopie en lumière non cohérente et qui comporte un support optoélectronique muni de plusieurs caméras balayant en une seule fois tout le champ dont l'empreinte doit être prise).

La caméra de prise d'empreinte optique peut utiliser des rayonnements photoniques (allant du bleu profond aux rayons X) ou ultrasoniques. Elle peut utiliser (ou non) des rayonnements cohérents.

Cette caméra peut également être associée à un dispositif d'interférométrie stéréoscopique ou à un projecteur projetant une lumière structurée (un point, une ligne, une grille...) sur un organe de la bouche (typiquement les dents ou la gencive).

Elle peut être associée à un système de mesure et d'analyse des formes des organes, le système ayant pour fonction de faire un relevé précis des formes et couleurs des organes. Dans le cas où le système de mesure et d'analyse est un projecteur de lumière active et/ou structurée, la caméra de prise d'empreinte optique possède au moins deux canaux (confondus ou séparés), l'un de projection et l'autre de reprise d'image. Le canal de projection (la projection pouvant être faite par LED, OLED, halogène, plasma ou laser) projette sur un organe un rayonnement structuré qui est déformé en fonction de la forme de la surface de l'organe, cette déformation étant transmise sur un capteur par le canal de reprise d'image. Ceci permet à la caméra de prise d'empreinte optique, par comparaison entre les caractéristiques de la lumière projetée (ou mémorisée) et la lumière déformée qui arrive sur le capteur, de connaitre la forme et les dimensions de chaque organe objet de l'analyse. La caméra de prise d'empreinte optique peut également être associée à un système de mesure et d'analyse des formes des organes de la bouche qui utilise des méthodes télémétriques ou stéréoscopiques mono ou multi-caméra (ce système de mesure a l'avantage d'être plus simple, mais les logiciels utilisés sont plus complexes). La caméra de prise d'empreinte optique peut également être associée à un système de mesure et d'analyse des formes des organes de la bouche formé par un instrument périphérique comme un appareil de radiologie, de tomographie en cohérence optique (OCT) ou d'émission ultrasonique (dans la mesure où ces appareils fournissent des informations métriques sur la zone étudiée).

Le dispositif selon l'invention permet également à l'utilisateur de savoir ce qui se passe dans la bouche du patient malgré la présence de la caméra de prise d'empreinte optique (notamment dans le cas où cette caméra est un porte-empreinte optique).

Le dispositif selon l'invention peut comprendre plusieurs caméras de prise d'empreinte optique. Chacune des caméras de prise d'empreinte optique peut être associée à un système de mesure et d'analyse.

Afin d'optimiser l'analyse et la prise d'empreinte, le dispositif selon l'invention peut être associé à un système d'éclairage qui peut fonctionner avec ou sans lumière structurée.

Ce système d'éclairage peut projeter depuis l'extérieur de la bouche ou être fixé sur la caméra de prise d'empreinte optique.

Le système d'éclairage, en fonction du type d'éclairage utilisé, peut faire apparaitre en réalité augmentée des informations supplémentaires sur les organes mesurés. Ainsi, suivant les longueurs d'onde de l'éclairage, il est possible de déterminer et/ou de retrouver certains éléments anatomiques dentaires et de les indiquer sur le verre optique.

Le système d'éclairage peut également être adapté à projeter certaines informations, sans avoir recours à la réalité augmentée. Ce peut être, par exemple, un indicatif (tel qu'un cercle rouge) projeté indiquant où se situe la lecture précise de la caméra de prise d'empreinte optique. Le système d'éclairage peut changer la couleur ou la forme de l'information en fonction de la qualité des données acquises.

La paire de lunettes à réalité augmentée comprend au moins un verre optique (en général, deux) et au moins une caméra de visualisation. La présence de plusieurs caméras de visualisation sur la paire de lunettes à réalité augmentée permet notamment de recaler en temps réel la modélisation issue de la lecture de la caméra de prise d'empreinte optique.

La caméra de visualisation n'est pas obligatoirement une caméra à trois dimensions : elle peut également être une caméra à deux dimensions ou à deux dimensions et demi. Le dispositif selon l'invention peut corréler des images prises en deux dimensions par une caméra de visualisation avec des images à trois dimensions prises par la caméra de prise d'empreinte. Dans le cas où la caméra de visualisation est une caméra à deux dimensions et demi ou une caméra à trois dimensions (la paire de lunettes disposant d'une vision spatiale), la corrélation est très exacte et les indications se font sur des parties des organes lues en trois dimensions (certaines paires de lunettes à réalité augmentée ont des verres optiques spécifiques dédiés rendant des résultats possibles).

Le verre optique au travers duquel l'utilisateur a une vision directe de la bouche est également le lieu où peuvent être projetées les premières images correspondantes à celles prises par la caméra de visualisation, les secondes images correspondantes à celles prises par la caméra de prise d'empreinte optique, et des éventuelles informations supplémentaires qui peuvent être associées à la réalité augmentée et à des instruments périphériques externes (par exemple un signe visualisable tel qu'une variation d'intensité, de couleur ou de contraste).

L'utilisateur peut ainsi voir l'évolution de son travail. Par exemple, il peut savoir si la prise d'empreinte optique est complète et précise ou s'il doit ré-intervenir pour la compléter, cette indication pouvant être réalisé par une substitution progressive de la vision directe au travers du verre optique par les secondes images représentant l'empreinte prise.

Les informations supplémentaires pouvant être projetées sur le verre optique (en temps réel ou en temps différé, au choix de l'utilisateur) peuvent être directement corrélées sur le verre optique comme le permet la réalité augmentée. Ces informations supplémentaires peuvent être issues d'instruments périphériques comme des appareils de radiologie, de tomographie en cohérence optique (OCT) ou d'émission ultrasonique. L'utilisation la réalité augmentée pour les informations supplémentaires permet de guider l'utilisateur (notamment si des organes n'ont pas été pris en empreinte), de lui montrer des informations sous-gingivales en utilisant des informations radiologiques préalablement mémorisées, de le mettre en garde durant un acte opératoire si ce dernier n'est pas parfaitement exécuté (par exemple, par l'indication de zones de contre-dépouille ou de mauvais parallélismes entre des piliers d'un bridge avant une prise d'empreinte définitive). Il est également possible d'associer les mouvements dynamiques lors de réalisation de coronoplastie ou d'analyses occlusales, de visualiser l'effet du positionnement prévu de brackets sur les dents, de connaitre la distribution des tissus dentaires durant la préparation de cavités destinées à recevoir une obturation ou une couronne (par exemple, la proximité de la pulpe).

La paire de lunettes à réalité augmentée utilisée peut être, par exemple, une paire de lunettes « Google Glass », « Vuzix smart Glass », « K-Glass » ou « video-projection Epson ».

Un système de repérage tridimensionnel peut être associé à la paire de lunettes à réalité augmenté afin que l'utilisateur puisse suivre la progression de son travail, et en particulier la progression de son action de mesure optique directement et en correspondance sur les organes de la bouche.

Le dispositif selon l'invention peut également comprendre une unité centrale de traitement informatique permettant notamment de corréler les premières images correspondantes à celles prises par la caméra de visualisation avec les secondes images correspondantes à celles prises par la caméra de prise d'empreinte optique.

Classiquement, l'unité centrale est adaptée à convertir des données analogiques et/ou digitales et à gérer des données (numériser les différentes images prises par la caméra de prise d'empreinte optique et la caméra de visualisation, mesurer différentes informations contenues dans ces images, traiter informatiquement ces images, les corréler, les réunir sur le verre optique...). L'unité central permet également d'ajouter des informations supplémentaires concernant le patient afin d'informer et d'aider l'utilisateur (par exemple, l'indication des organes dont la mesure a été prise).

L'unité centrale permet de réunir en un même objet les premières images avec les secondes images. Ces premières et secondes images étant prises d'une même zone de travail mais présentent des informations distinctes. En fonction des circonstances, l'unité centrale substitue ou superpose les secondes images aux premières images.

La corrélation suivie d'une substitution entre les deux types d'images de la même zone de travail se traduit par un enrichissement progressif de la vue directe de l'utilisateur par les secondes images numérisées et traitées. Par exemple, chaque organe situé dans la bouche présente des particularités visuelles auxquelles est associé un nuage de points. L'unité centrale, d'une part, retrouve le même nuage de points sur les premières et les secondes images (généralement, concernant les secondes images, au fur et à mesure que ces secondes images sont prises) et, d'autre part, corrèle les deux types d'images en affichant les secondes images qui sont beaucoup plus précises que les premières images. Cet affichage de corrélation peut être réalisé dans une couleur particulière. L'utilisateur voit ainsi apparaître directement et progressivement sur le verre optique la zone mesurée (par exemple coloriée en rouge), et ceci même si cette zone est cachée par la caméra de prise d'empreinte optique (notamment dans le cas où cette caméra est un porte-empreinte optique), les secondes images étant justement prises par la caméra de prise d'empreinte optique.

L'unité centrale peut également avoir d'autres fonctions, par exemple permettre de calculer, pour un organe, un nuage de points en trois dimensions spatiales, y ajouter le temps des mouvements de l'organe (quatrième dimension) et sa teinte (cinquième dimension).

L'unité centrale peut comprendre un logiciel de traitement d'images muni de plusieurs algorithmes. Ainsi, un flux d'images en provenance d'une ou de deux caméras à deux dimensions est traité en temps réel de manière à produire une première reconstruction en trois dimensions en temps réel visualisable par l'utilisateur au fur et à mesure que celui-ci déplace la caméra de prise d'empreinte optique aux alentours de l'organe. Le schéma global de reconstruction en trois dimensions en temps réel et l'organisation des données varient en fonction du nombre de caméra utilisée : soit deux flux d'images à deux dimensions, chacun de ces flux d'images provenant d'une caméra à deux dimensions, soit un flux d'image à deux dimensions provenant d'une unique caméra à deux dimensions associé à un flux de données d'accélération provenant d'un accéléromètre. Chaque image nouvellement acquise est traitée par un algorithme de recherche de trace optique par calcul des points d'intérêt et par mise en correspondance au travers des images. A partir des correspondances, un algorithme de séquençage en temps réel met alors à jour le séquençage du flux vidéo pour une meilleure performance temporelle. Un algorithme d'estimation parallèle des positions des caméras dans l'espace et des coordonnées des points en trois dimensions permet ensuite, grâce aux traces optiques, de retrouver les positions des caméras au moment de l'acquisition et de générer le nuage de points en trois dimensions se projetant sur les traces optiques. Le nuage de points généré est ensuite interpolé par un algorithme d'interpolation pour obtenir un nuage plus dense. Est également calculée une fonction implicite d'interpolation grâce à laquelle est obtenue, par algorithme de polygonalisation et de calcul de texture, une polygonalisation texturée de la surface à reconstruire. A cette étape, il est possible de calculer des indices de qualité du nuage de points final et éventuellement d'étiqueter certains points comme non valides. La surface texturée est affichée, avec éventuellement des annotations adaptées pour indiquer les zones encore non valides. Cette surface générée en temps réel est une représentation de la zone reconstruite à un facteur d'échelle. Lorsque l'acquisition est terminée, le facteur d'échelle est calculé par un algorithme de mise à l'échelle de la reconstruction en trois dimensions. Enfin, un algorithme de rehaussement de précision spatiale peut être utilisé sur le modèle en trois dimensions de manière à avoir une reconstruction la plus fidèle possible. Cet algorithme de rehaussement recalcule un nuage de points en trois dimensions en tenant compte de l'ensemble des vues acquises. Le nuage recalculé est ensuite interpolé par l'algorithme d'interpolation, et enfin un modèle global en trois dimensions est reconstruit par un algorithme de « space carving ».

Le dispositif selon l'invention peut également être associé à des instruments périphériques fournissant des informations supplémentaires telles que des informations radiographiques, colorimétriques, ou de mouvement mandibulaires.

Ces instruments périphériques sont de préférence associés à l'unité centrale, comme le sont la caméra de prise d'empreinte optique et la caméra de visualisation, de sorte que l'unité centrale puisse traiter les informations fournies par ces instruments périphériques et projeter des images supplémentaires correspondantes sur le verre optique de la paire de lunettes à réalité augmentée.

Un instrument périphérique possible est une caméra exobuccale adaptée à prendre une partie du visage du patient sur un champ large. Cette caméra est notamment utilisée pour certains traitements comme en orthodontie ou en implantologie. A cette caméra exobuccale peuvent être associées les fonctionnalités que celles décrites pour la caméra de prise d'empreinte optique ou pour la caméra de visualisation (notamment, l'utilisation ou non de lumière structurée).

Un autre instrument périphérique possible est une caméra à deux dimensions adaptée à prendre des images dont les informations à extraire ne nécessitent pas forcément d'être d'une grande précision, comme, par exemple, la teinte des dents, la présence de saignements, l'atteinte des tissus durs (la présence de caries), la présence de tumeurs ou de certaines pathologies buccales.

D'autres instruments périphériques possibles sont des instruments de mesure de position, tels que des accéléromètres, gyromètres ou magnétomètres. Ces instruments de mesure de position peuvent être portés aussi bien par la caméra de prise d'empreinte que par la paire de lunettes à réalité (pour la caméra de visualisation). Ces instruments de mesure de position permettent de mesurer la position de l'outillage les portant et de faciliter la corrélation des images prises. Ils peuvent également suppléer à une éventuelle panne de l'un des capteurs sur l'une ou l'autre des caméras.

Parmi les instruments périphériques, il est également possible d'avoir un articulateur, des jauges de pression et un occluseur numérique. Ces instruments permettent de visualiser, d'analyser et d'incorporer aux images vues au travers du verre optique les mouvements du corps du patient, comme les mouvements mandibulaires ou la réponse à l'impression de certaines images pathologiques sur la gencive.

Un autre instrument périphérique possible est un colorimètre ou un spectro-colorimètre permettant de connaître la couleur des dents, que ce soit d'une manière absolue ou d'une manière relative en référence à des couleurs connues sur des teintiers mémorisés.

Il est également possible d'avoir des instruments périphériques permettant de connaître l'état de santé du patient durant l'acte opératoire (par exemple, la pression et l'oxygénation sanguine, la température du corps, la température de la lésion étudiée...) .

Il est également possible d'avoir des instruments périphériques dentaires (par exemple des appareils radiologiques, ultrasoniques, magnétiques (IRM), térahertzien...) permettant de prendre et enregistrer des images des organes de la bouche.

Il est également possible d'avoir des instruments périphériques apportant à l'utilisateur des informations complémentaires permettant de l'aider lors de son travail (ces informations peuvent être issues de fonctions mémorisées ou de logiciels intra ou extra muros, par exemple, par télémédecine).

Il est également possible d'avoir des écrans déportés permettant l'affichage des informations que l'utilisateur voit sur le verre optique et permettant à des assistants ou à des élèves de suivre les travaux effectués.

Il est également possible d'avoir une machine-outil à commande numérique pouvant réaliser une pièce réelle correspondant à l'image vue par l'utilisateur.

Il est également possible d'avoir un micro (par exemple incorporé à une branche de la paire de lunettes) permettant à l'utilisateur de donner des ordres à l'unité centrale (par exemple pour l'apparition ou la disparition d'informations sur le verre optique).

Il est également possible d'avoir tout autre instrument périphérique permettant de communiquer avec l'utilisateur, les assistants, les étudiants et/ou l'unité centrale, ou permettant de transmettre et de stocker les informations, les ordres et les données.

L'ensemble des éléments constitutifs du dispositif selon l'invention peuvent être reliés entre eux par câble, par Bluetooth ou par Wifi (ces deux derniers cas libérant complètement les gestes de l'utilisateur).

Le dispositif selon l'invention est simple dans sa fabrication, ce qui le rend particulièrement résistant et peu onéreux. Il peut être utilisé dans les cabinets dentaires et les laboratoires de prothèse. Il permet à l'utilisateur de travailler de manière rapide, sécurisée et confortable. Il permet notamment de réaliser des empreintes optiques en dentisterie dans des conditions optimales. Il permet également d'optimiser l'action thérapeutique en offrant une précision de l'ordre du micron.

Le dispositif peut être appliqué, dans une forme évolutive, à tout système d'acquisition en trois dimensions nécessitant une manipulation rapide et précise obligeant l'utilisateur à ne pas quitter des yeux son champ de travail, d'analyse et/ou de mesure (tel que dans les milieux médicaux, sportifs ou industriels). Il est ainsi possible de suivre et informer l'utilisateur en temps réel (ou quasi réel) tout en lui permettant de ne pas quitter la scène des yeux et en lui affichant des informations.

D'autres particularités et avantages de la présente invention apparaîtront dans la description d'un mode de réalisation du dispositif de visualisation de l'intérieur d'une bouche d'un patient, ce mode de réalisation étant donné à titre d'exemple non limitatif et représenté par les dessins mis en annexe dans lesquels :
- La figure 1 est une représentation schématique du dispositif conforme à la présente invention ;
- La figure 2 est une autre représentation du dispositif conforme à la présente invention ;
- La figure 3 est une représentation de l'organisation des éléments essentiels au dispositif conforme à la présente invention ;
- La figure 4 est une représentation des étapes de mise en œuvre du dispositif conforme à la présente invention ;
- La figure 5 est une vue d'une arcade dentaire au travers d'un verre optique du dispositif conforme à la présente invention ;
- La figure 6 est une vue de la modélisation de l'arcade dentaire de la figure 5 après prise d'images par une caméra de prise d'empreinte optique endobuccale du dispositif conforme à la présente invention ;
- La figure 7 est une vue, au travers du verre optique, de l'arcade dentaire de la figure 5, la partie de l'arcade dont l'empreinte a été prise étant substituée par la modélisation précise de cette partie d'arcade ;
- La figure 8 est une vue d'une série de dents au travers d'un verre optique ;
- La figure 9 est une vue représentant une mise en correspondance automatique par logiciel des points homologues de la vue de la figure 8 avec un nuage de points ;
- La figure 10 est une vue représentant une mise en correspondance automatique par logiciel des points homologues de la vue de la figure 8 avec une modélisation filaire ;
- La figure 11 est une représentation similaire à celle de la figure 3, mais représentant également des éléments constitutifs accessoires du dispositif conforme à la présente invention ;
- La figure 12 est une représentation des étapes permettant de visualiser et d'analyser l'occlusion statique et dynamique d'un patient par le dispositif conforme à la présente invention ;
- La figure 13 est une vue des zones en contact statique déterminées par le dispositif conforme à la présente invention ;
- La figure 14 est une vue des zones en contact dynamique déterminées par le dispositif conforme à la présente invention ;
- La figure 15 est un exemple de représentation de ce qui est projeté sur le verre optique afin de permettre de suivre en temps réel les mouvements dynamiques de l'occlusion du patient ;
- La figure 16 représente une prise d'empreinte faite à l'aide de la caméra de prise d'empreinte endobuccale avec, en superposition, la forme de la préparation idéale et l'axe d'insertion de la préparation idéale comparée à l'axe d'insertion de la préparation réalisée ;
- La figure 17 représente une future prothèse réalisée à l'aide de logiciels de modélisation dentaire, vue en superposition sur la prise d'empreinte réalisée à l'aide de la caméra de prise d'empreinte endobuccale ;
- La figure 18 est une représentation en couleur d'une arcade complète réalisée avec la caméra de prise d'empreinte endobuccale ;
- La figure 19 est une représentation d'un écran visible au travers du verre optique de la paire de lunettes à réalité augmentée, écran où se trouvent affichées les informations colorimétriques ou spectrocolorimétriques en fonction des zones concernées ; et
- La figure 20 est une représentation de la prise d'empreinte réalisée à l'aide de la caméra endobuccale à laquelle est associée une représentation des lèvres et de la joue, affichée et visible au travers du verre optique de la paire de lunettes à réalité augmentée.

Les présentes figures représentent différentes mises en œuvre du dispositif de visualisation 1 de l'intérieur d'une bouche d'un patient 2 conforme à la présente invention en montrant toutes les possibilités qu'il offre dans la pratique quotidienne d'un utilisateur 3, par exemple un chirurgien-dentiste. Ce dispositif 1 trouve un intérêt tout particulier dans les domaines de la dentisterie.

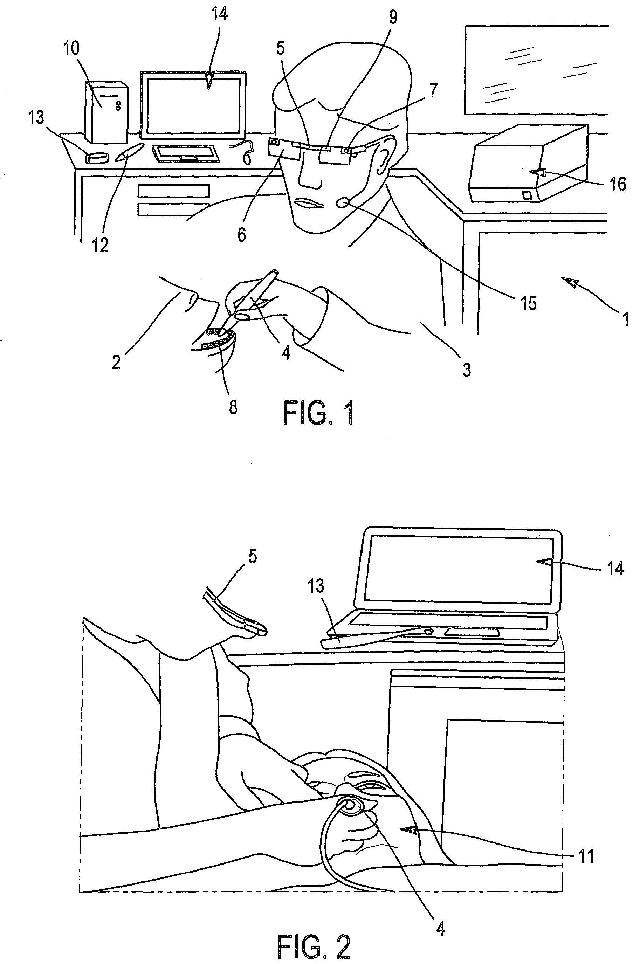

La figure 1 représente des éléments du dispositif de visualisation 1, la visualisation étant enrichie grâce au procédé de réalité augmentée. Le dispositif de visualisation 1 permet à l'utilisateur 3 de ne jamais quitter son champ opératoire lorsqu'il réalise ses mesures ou ses diagnostics.

Le dispositif 1 comprend une caméra de prise d'empreinte optique 4. En l'occurrence, cette caméra de prise d'empreinte optique 4 est une caméra endobuccale manuelle permettant à l'utilisateur 3 (dentiste ou médecin) d'effectuer ses mesures en trois dimensions dans la bouche ou sur la peau de son patient 2. Cette mesure étant très précise (quelques microns) et très proche des dents, la profondeur de champs est très faible, ce qui explique que l'utilisateur 3 doit procéder à un balayage de l'ensemble des dents, soit par photos successives (one shoot impression), soit par filmage en trois dimensions (full motion). La caméra de prise d'empreinte optique 4 pourrait être un porte-empreinte optique, tel que décrit dans le brevet US 8 520 925, renfermant plusieurs caméras et permettant à l'utilisateur 3 d'avoir en une seule fois l'ensemble de la bouche sans être obligé de la balayer.

Le dispositif 1 comprend également une paire de lunettes à réalité augmentée 5 qui est portée par l'utilisateur 3. Cette paire de lunettes à réalité augmentée 5 comprend deux verres optiques 6 et deux caméras de visualisation 7 stéréoscopiques. En conséquence, elle permet à l'utilisateur 3 d'avoir une vision naturelle stéréoscopique de la bouche 8 du patient 2 et donc de la zone qu'il mesure et qu'il étudie. Lorsque l'utilisateur 3 regarde cette zone de travail, les caméras de visualisation 7 observent la même scène et procèdent à un relevé d'information conduisant à la création d'un nuage de points de visualisation.

Comme la tête de l'utilisateur 3 peut bouger par rapport à la zone observée, il a été adjoint à la paire de lunettes à réalité augmentée 5, à proximité des verres optiques 6, des dispositifs 9 facilitant le suivi dans l'espace l'axe d'observation de l'utilisateur 3 (accéléromètre / gyromètre / magnétomètre à trois dimensions). Bien que non obligatoire, cette adjonction facilite grandement le travail lorsque l'utilisateur 3 doit déplacer son regard en dehors de la zone de travail et ensuite y revenir pour continuer son travail.

Le dispositif 1 comprend également une unité centrale 10 qui permet de traiter les images prises par la caméra de prise d'empreinte optique 4 et celles prises par la caméra de visualisation 7.

Les mesures prises par la caméra de prise d'empreinte optique 4 et celles prises par la caméra de visualisation 7 fournissent deux fichiers correspondant à une même zone mais n'ayant pas la même précision. Ces fichiers peuvent être de simples informations électro-optiques ou des informations plus sophistiquées comme des représentations numériques sous forme de nuages de points ou même de modélisations surfaciques ou volumiques. Dans tous les cas, il existe entre ces deux fichiers des valeurs communes comme par exemple les points se situant dans des zones de référence facilement identifiables comme le sommet des cuspides des dents ou le fond de leurs sillons. Ces valeurs communes (par exemple les zones de référence) permettent à l'unité centrale 10 de fusionner et/ou de superposer les deux fichiers en un seul tout en leur préservant leurs spécificités.

Le dispositif 1 comprend également un système d'éclairage 11 qui peut faciliter la lecture en trois dimensions des dents qui ont une réflexion très spéculaire. Le système d'éclairage 11 émet une lumière spécifique pouvant être une projection active et structurée comme par exemple la projection de grilles ou autres motif. Il serait aussi possible d'utiliser une caméra de prise d'empreinte optique 4 n'utilisant pas de lumière structurée mais s'appuyant sur les principes de la stéréoscopie passive (AWS ou autre), sur la technique comme le temps de vol, ou sur des techniques holographiques ou leurs dérivées comme la tomographie en cohérence optique (OCT).

Le nouveau dispositif de visualisation 1 est totalement universel et applicable à toute forme de mesures localisées endobuccales. A la différence des techniques architecturale utilisées classiquement par des paires de lunettes à réalité augmentée qui recherchent des points spécifiques, le dispositif 1 selon l'invention utilise une double empreinte optique (celle issue de la caméra de prise d'empreinte optique 4 et celle issue (en même temps ou en temps différé) par la caméra de visualisation 7 portée par la paire de lunettes à réalité augmentées 5) et les enrichit et/ou les substitue en fonction de leur degré de précision.

Le dispositif 1 comprend également des instruments périphériques qui sont reliés à l'unité centrale 10 afin de pouvoir adjoindre au fichier de mesure constitué par exemple d'un nuage de points d'autres fichiers complémentaires (d'autres nuages de points), comme des informations radiologiques ou ultrasoniques ou même des informations volumétriques exobuccales obtenus à l'aide d'une caméra à deux dimensions 12 ou à d'une caméra à large champ 13.

L'utilisateur 3 peut aussi exporter ces données pour les visualiser sur un écran déporté 14 (également visible par ses assistants) ou sur son unité centrale 10, l'utilisateur 3 communiquant à l'aide d'un micro 15 indépendant de la paire de lunettes 5 ou fixé sur cette dernière. Il peut encore les exploiter ces données pour faire, via une machine-outil 16, un usinage rapide en cours de travail lui permettant de mieux comprendre l'environnement immédiat lors de la préparation des dents qui vont recevoir la prothèse. Cet usinage peut se faire par soustraction (usinage conventionnel par fraisage) ou par addition (usinage non conventionnel comme la fusion laser ou la stéréo lithographie).

Il va de soi que dans le cas de l'utilisation d'un porte-empreinte optique, toute l'arcade serait recouverte et le clinicien ne serait pas dérangé par l'éclairage utilisé (continu ou pulsatile).

A la figure 2 est représentée l'invention qui comprend une caméra de prise d'empreinte endobuccale 4 en stéréoscopie passive associée à un système d'éclairage 11 projetant un éclairage spécifique pour mesurer les dents et la gencive (lumière blanche à dominante bleue). L'unité centrale 10 qui fait partie d'un ordinateur portable, est puissante et conventionnelle. Le même ordinateur portable comprend un écran 14 utilisable par un assistant.

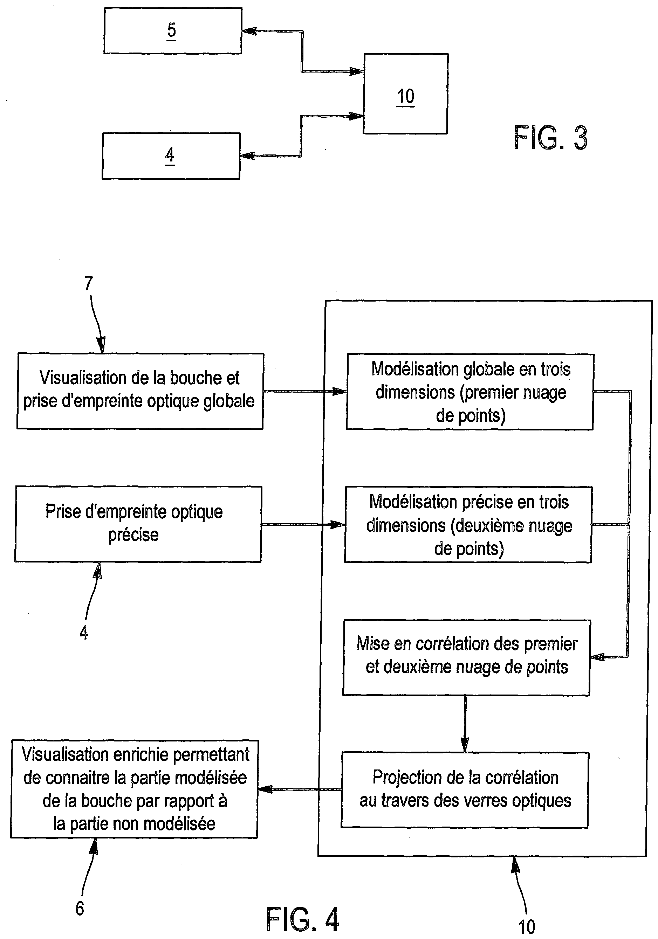

A la figure 3 est représenté l'agencement des éléments essentiels au dispositif 1 conforme à l'invention : une caméra de prise d'empreinte optique 4 (caméra endobuccale ou porte-empreinte optique) pour lire précisément les dents, la gencive, la peau..., une paire de lunettes à réalité augmentée 5 permettant à l'utilisateur 3 de voir en un unique champ le champ opératoire (en vision directe) et la modélisation très précise réalisée par la caméra de prise d'empreinte optique 4 (qui s'y affiche progressivement au fur et à mesure de sa réalisation), et une unité centrale 10 qui referme les programmes et les données mémorisées.

La figure 4 illustre les différentes étapes de mise en correspondance de la vue prise par la paire de lunettes à réalité augmentée 5 avec la vue prise à l'aide de la caméra de prise d'empreinte optique 4.

L'utilisateur 3 observe le champ de travail dans la bouche 8 de son patient 2 directement au travers des verres optiques 6 de sa paire de lunettes à réalité augmentée 5, les deux caméras de visualisation 7 fixées sur cette paire de lunettes 5 effectuant un relevé d'un premier nuage de points permettant à l'unité centrale 10 de modéliser l'ensemble de la surface dans un référentiel connu et métrique général mais insuffisamment précis pour réaliser une prothèse dentaire (en l'occurrence, l'unité centrale 10 utilise le principe de la stéréoscopie). Plus il y aura de caméras de visualisation 7, plus le relevé sera précis, plus le nombre de points relevés sera élevé, et plus la corrélation avec les images issues de la caméra de prise d'empreinte optique 4 sera précise.

Une fois que les caméras de visualisation 7 ont fini de faire le relevé, l'utilisateur 3 prend alors la caméra de prise d'empreinte optique 4 à la main et effectue un balayage précis des dents, de la gencive ou éventuellement de la peau conduisant à la création d'un deuxième nuage de points proche mais beaucoup plus précis que celui obtenu à l'aide des caméras de visualisation 7, la caméra de prise d'empreinte optique 4 ayant une plus forte densité, et donc une plus forte précision, que les caméras de visualisation 7. Si la caméra de prise d'empreinte optique 4 est un porte-empreinte optique tel que décrit dans le brevet US 8 520 925, le relevé peut de faire sans balayage.

Pendant la lecture par la caméra de prise d'empreinte optique 4, l'unité centrale 10 reconnait et corrèle les deux nuages de points obtenus (celui issu des deux caméras de visualisation 7 et, au fur et à mesure du balayage, celui issu de la caméra de prise d'empreinte optique 4). Cette double modélisation apparait sur les verres optiques 6 de la paire de lunettes à réalité augmentée 5. L'utilisateur 6 n'a plus à suivre son travail sur un écran déporté 14. Il verra se construire, grâce au dispositif de visualisation 1, directement sur les dents de son patient 2, les zones mesurées et celles qui ne le sont pas encore.

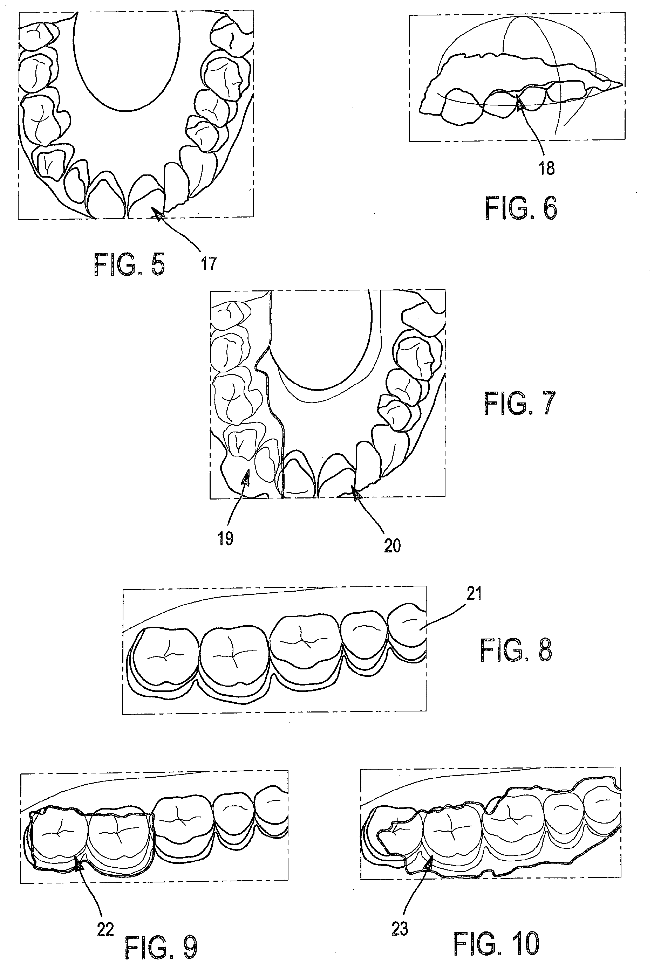

Les figures 5 à 7 illustrent plus précisément ces étapes. La figure 5 représente une arcade dentaire 17 qui est vue par l'utilisateur 3 et par les caméras de visualisation 7, ces dernières effectuant le relevé du premier nuage de points de l'arcade 17. La modélisation correspondante est ici en trois dimensions, des vues en deux dimensions ou en deux dimensions et demi seraient suffisantes. Une fois le relevé du premier nuage de points terminé, l'utilisateur 3 prend la caméra de prise d'empreinte optique 4 et commence à effectuer le relevé précis des organes (dents, gencive, peau) et obtient une modélisation précise 18 en vue vestibulaire à trois dimensions d'une partie de l'arcade 17 (figure 6). L'unité centrale 10 fait correspondre les deux nuages de points dans les verres optiques 6 portées par l'utilisateur 3. Ce dernier voit apparaitre au fur et à mesure de son travail avec la caméra de prise d'empreinte optique 4 se substituer ou se superposer la modélisation précise 18 sur la totalité de l'arcade 17 qui est à mesurer avec précision. Comme illustré à la figure 7, la vision directe de l'arcade 17 est donc augmentée par l'indication des zones mesurées 19 (correspondantes à la modélisation précise 18) par rapport à celles qui ne le sont pas 20, ce qui permet à l'utilisateur de contrôler l'avancement de son travail.

Grâce à la réalité augmentée, et en fonction de la qualité des informations recueillies par la caméra de prise d'empreinte optique 4, il est possible de superposer différentes informations supplémentaires. Ainsi, la qualité de la précision de la prise de vue faite par la caméra de prise d'empreinte optique 4 peut être associée à des informations supplémentaires (par exemple une couleur rouge si la qualité est insuffisante, verte si elle est parfaite), l'utilisateur 3 gardant le choix de continuer son balayage avec la caméra 4 ou de revenir sur une zone de qualité insuffisante. De même, les informations supplémentaires peuvent être signes particuliers (par exemple une flèche) indiquant une zone particulière ou rappelant une action particulière à effectuer.

Les figures 8, 9 et 10 permettent de voir des réalisations de corrélation. La figure 8 représente un modèle réel 21. La figure 9 représente une corrélation, sur les deux dernières molaires, d'un nuage de points 22 issu d'une caméra de prise d'empreinte optique 4. La figure 10 représente une corrélation à partir d'une modélisation filaire 23.

Par l'invention, l'utilisateur 3 a une empreinte optique très précise (apportée par la caméra de prise d'empreinte optique 4) corrélée et venant enrichir la vue imprécise qui lui donnée par les caméras de visualisation 7. L'utilisateur 3 voit ainsi un objet réel relativement imprécis tout en pouvant travailler sur un nouvel environnement virtuel, d'une manière totalement transparente, en bénéficiant de la haute précision apportée par la caméra de prise d'empreinte optique 4.

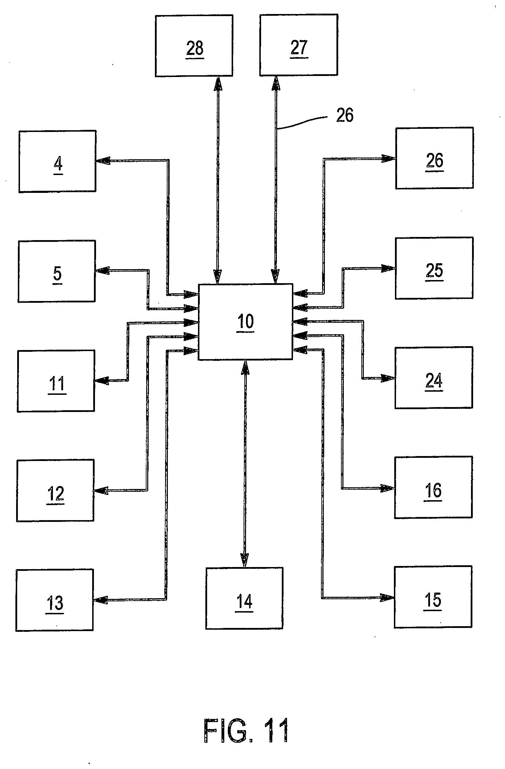

La figure 11 montre une partie des possibilités offertes par les instruments périphériques associés au dispositif 1 de la présente invention dans le domaine de la dentisterie.

Toutes les informations supplémentaire issues des instruments périphériques (caméras externes 12 ,13, capteurs 24 de données dynamiques de mouvement, colorimètres 25 permettant de capter les couleurs présentes dans la bouche 8, capteurs médicaux 26 analysant les état physiologiques du patient 2, instruments 27 analysant des données radiologiques arrivant en temps réel ou en temps différé, instruments 28 permettant la connexion des données externes arrivant par les voies de la télémédecine ou en stockage dans l'unité centrale 10) sont reportées sur une modélisation précise obtenue en haute définition par la caméra de prise d'empreinte optique 4 corrélée sur la vision directe moins précise mais directement observée par l'utilisateur 3 dans son champ de travail.

Le geste de l'utilisateur 3 sera d'autant plus libre et sa vision directe que les connexions 29 entre les différents composants du dispositif 1 se fera par un long câble ou sans câble (Wifi, Bluetooth...) . Si les connexions 29 se font par câbles, elles se feront de préférence par une connexion USB autoalimentée. Avantageusement, les connexions 29 se font sans câble.

L'utilisateur 3 peut recevoir des informations supplémentaires statiques et dynamiques en suivant les mouvements dans le temps des variations de la vue enrichie par la caméra de prise d'empreinte optique 4 et visualisée en réalité augmentée au travers des verres optique 6.

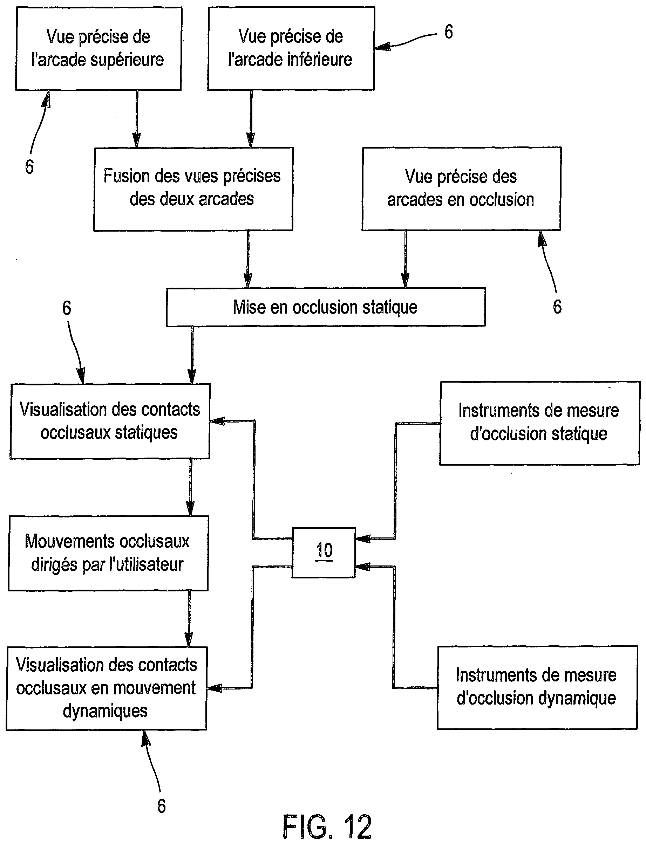

La figure 12 illustre le traitement des informations supplémentaires dynamiques, notamment pour les mouvements d'occlusion (les contacts occlusaux étant les zones ou les dents du haut et celle du bas se touchent).

En premier lieu, l'utilisateur 3 fait une empreinte optique du maxillaire supérieur puis de la mandibule inférieure du patient 2 par la caméra de prise d'empreinte optique 4 et peut visualiser chacune des deux vues via la paire de lunettes à réalité augmentée 5. L'utilisateur 3 demande ensuite au patient 2 de serrer les dents en occlusion clinique statique thérapeutique ou de convenance et reprend la caméra de prise d'empreinte optique 4 afin d'avoir une prise d'empreinte optique précise vestibulaire en occlusion et une visualisation en occlusion. Celle-ci se substitue ou se superpose à la vue générale observée au travers de la paire de lunettes à réalité augmentée 5. L'utilisateur 3 a ainsi une vue réunissant les deux arcades du patient selon les principes cliniques de l'occlusion clinique.

L'utilisateur 3 peut suivre dans le temps les déplacements du modèle de la mandibule par rapport au modèle du maxillaire, et peut ainsi déterminer les contacts occlusaux.

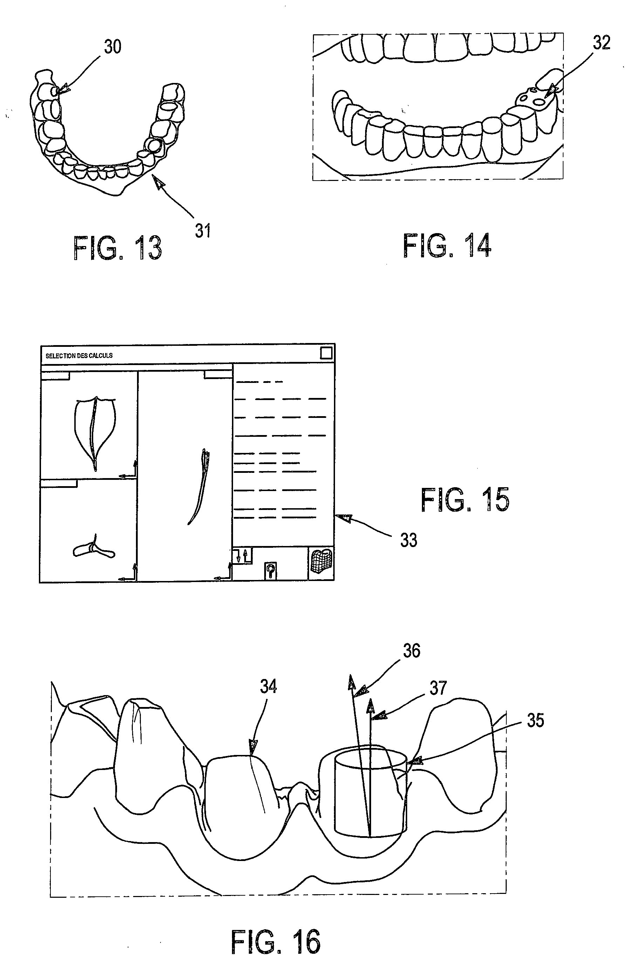

Si l'utilisateur 3 met entre les dents du patient 2 des moyens de mesure de pression et de surfaces de pression (par exemple, des jauges de pression) au moment où ce dernier serre les dents, il peut obtenir une valeur de la pression exercée et de la surface impliquée à chaque niveau des contacts occlusaux. La figure 13 représente une vue de ces contacts occlusaux 30 projetés sur une arcade modélisée 31. Ces valeurs peuvent aussi être mesurées par la caméra de prise d'empreinte optique 4 de façon très précise (à quelques microns près), au moment où le patient 2 serre les dents.

La figure 14 illustre une représentation sur un verre optique 6, des contacts occlusaux dits prématurés 32 obtenus après que l'utilisateur 3 ait demandé au patient 2 de déplacer sa mandibule suivant des mouvements occlusaux. Là encore, l'utilisateur 3 du dispositif de visualisation 1 peut suivre ces mouvements et l'apparition de ces contacts 32 directement dans la bouche du patient 2 (contacts plaqués sur les dents naturelles), sans à avoir à détourner son regard hors du champ de travail.

Si l'utilisateur 3 utilise un dispositif d'analyse mandibulaire 33 tel qu'illustré à la figure 15, il peut suivre les mouvements mandibulaires directement dans la bouche du patient 2 et avoir les trajets d'occlusion, les déterminants antérieurs et postérieurs et l'indication des contacts nécessitant d'éventuelles corrections. Il suffit d'indexer en temps réel le facteur temps/mouvement sur le nuage de points ou sur la modélisation. De même ce facteur temps/mouvement peut être utilisé par l'unité centrale 10 en temps différé si l'utilisateur 3 souhaite observer ces mouvements postérieurement dans le but de faire une analyse de l'évolution de la pathologie ou de la thérapeutique occlusale (via une corrélation prenant comme référence des points communs tels que des sillons sur les dents ou une structure de la gencive).

Comme illustré à la figure 16, l'utilisateur 3 peut voir, durant son travail de préparation d'un site de réception d'une reconstitution dentaire 34, des indications partielles qui sont directement reportées sur la modélisation précise qui est confondue avec les dents naturelles du patient 2. Ces indications peuvent être de différentes formes (flèches, surimposition colorées pour les contredépouilles, etc.). En particulier, l'utilisateur 3 peut contrôler, directement dans la bouche 8 du patient 2 si les critères de forme et d'espace de la reconstitution sont suffisamment respectés. Le dispositif 1 selon l'invention permet de projeter sur les dents en préparation (au travers des verres optiques 6) de façon beaucoup plus précise (grâce à la caméra de prise d'empreinte optique 4), ce que doit être une bonne préparation 35 ou un bon axe de parallélisme 36 pour un bridge ou un implant par rapport à ce que cet axe est en temps réel en cours du travail 37. Cela permet à l'utilisateur 3 de contrôler son travaille au fur et à mesure qu'il l'effectue (il est possible d'avoir une visualisation de l'intrados idéale avec les parties dentaires non encore correctement taillées signifiées par un visuel spécifique, par exemple, surlignées en une couleur).

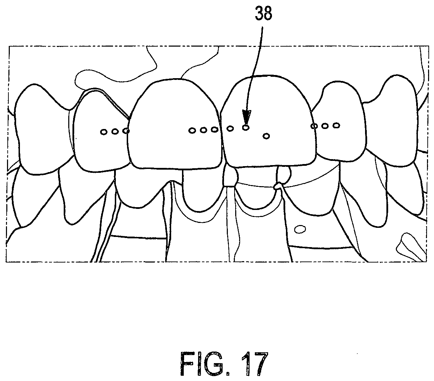

Comme illustré à la figure 17, il est possible, après avoir réalisé la préparation dentaire, de projeter et de visualiser directement sur l'arcade du patient 2 la modélisation finale 38 de la prothèse (avant de l'avoir faite). Il est ainsi de bâtir une modélisation de la surface externe de la prothèse (sous forme surfacique ou volumique) directement dans la bouche 8 du patient 2. Il est même possible d'agir sur la forme modélisée de la prothèse (en s'appuyant sur la modélisation issue de la réalité augmentée et les mesures faites avec la caméra de prise d'empreinte optique 4) pour que la prothèse réelle soit parfaitement adaptée à l'anatomie, à la physiologie et à l'esthétique du patient 2.

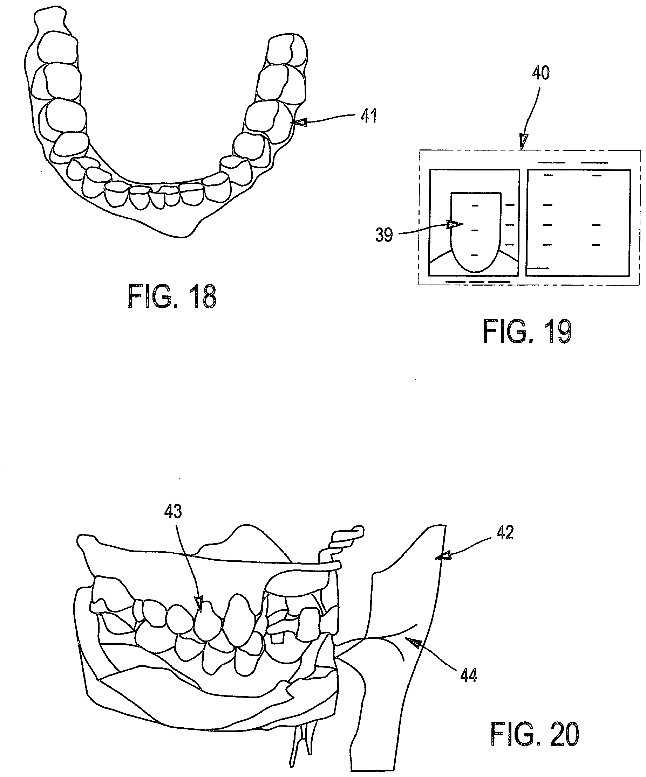

Comme illustré à la figure 18, l'utilisateur 3 peut voir directement sur la zone de travail la concordance entre la couleur choisie d'une prothèse modélisée et les couleurs des organes de la bouche 8 du patient 2.

De façon plus précise, les étapes de détermination de la couleur de la prothèse modélisée peuvent être les suivantes. Dans un premier temps, une modélisation extérieure sans couleur de la prothèse est réalisée et projetée sur les verres optiques 6 (si nécessaire, l'utilisateur 3 corrige la forme modélisée de la prothèse en fonction notamment de critères fonctionnels, esthétiques et thérapeutiques). Dans un deuxième temps, l'utilisateur 3 analyse la couleur des dents naturelles (soit manuellement à l'aide d'un teintier, soit à l'aide d'un appareil du type colorimètre ou spectro-colorimètres, soit à l'aide d'une caméra endobuccale si celle-ci utilise des dispositifs à transfert de charge (CCD) pour la couleur ou pour le noir et blanc avec possibilité de déduire les couleurs grâce à un calibrage approprié), l'analyse pouvant se faire localement (par segmentation de la dent 39, par exemple, en la divisant en un collet, un centre et un bord de dent), comme illustré à la figure 19, ou de manière générale à partir d'une couleur moyenne obtenue sur toute la surface de la dent. Dans un troisième temps, l'utilisateur 3 demande une projection de la prothèse modélisée associée à la couleur choisie. Dans un dernier temps, à l'aide d'un teintier virtuel 40 tel que celui de la figure 19, l'utilisateur 3 peut modifier la couleur de la prothèse modélisée soit sur toute la surface, soit dans des zones précises, et voir les conséquences de ces modifications sur l'intégration de la prothèse modélisée directement dans la bouche 8 du patient 2 (ainsi, des zones sombres 41 à modifier apparaissent à la figure 18) avant de lancer la fabrication de la prothèse.

La précision de la position des couleurs n'étant pas aussi importante que celle nécessaire à la réalisation de la forme de la prothèse, il peut être possible que la précision des caméras de visualisation 7 de la paire de lunettes à réalité augmentée 5 soit suffisante.

Comme illustré à la figure 20, le dispositif 1 conforme à la présente invention peut comprendre une caméra à large champ (comme une caméra exobuccale) permettant de prendre des images d'au moins une partie du visage 42 du patient 2. L'unité centrale 10 peut corréler ces images du visage avec l'ensemble des applications précédemment décrites (par exemple, la modélisation 43 des organes et des prothèses), notamment en utilisant des composantes esthétiques générales du patient 2 telles que la ligne du sourire, le plan de Camper ou la position des commissures des lèvres 44 (ces composantes, retrouvées par l'unité centrale 10, peuvent être affichées sur les verres optiques 6 de la paire de lunettes à réalité augmentée 5). L'analyse de la position des composantes esthétiques générales du patient 2 ne nécessitant pas une grande précision, elle peut être réalisée à partir d'images en deux dimensions ou en deux dimensions et demi prises par un instrument périphérique plus simple et moins cher qu'une caméra de prise d'empreinte optique 4, comme une caméra exobuccale ordinaire.

L'utilisateur 3 peut ainsi affiner l'esthétique de la future prothèse directement sur le visage du patient 2, via la paire de lunettes à réalité augmentée 5.

Enfin, l'unité centrale 10 peut être connectée à des informations extérieures grâce à un réseau de télémédecine, et peut ainsi commander l'affichage d'informations en temps réel sur des images nécessitant certaines expertises (préparation) ou un réseau interne déporté destiné aux assistants ou aux élèves dans un centre d'enseignement.

## Revendications

1. Dispositif de visualisation (1) de l'intérieur d'une bouche (8) d'un patient (2), le dispositif (1) comprenant, premièrement, une paire de lunettes ayant un verre optique (6) au travers duquel un utilisateur (3) de la paire de lunettes peut voir l'intérieur de la bouche (8), deuxièmement, une caméra **endobuccale** (4) adaptée à prendre une empreinte optique d'organes disposés dans la bouche (8), et, troisièmement, une unité centrale (10) adaptée à projeter sur le verre optique (6) des premières images correspondantes à celles prises par la caméra de prise d'empreinte optique (4), **caractérisé en ce que** ladite paire de lunettes est à réalité augmentée (5) comprenant une caméra de visualisation (7) adaptée à prendre en image ce que l'utilisateur (3) voit au travers du verre optique (6), ladite unité centrale (10) étant adaptée :
- à mesurer des informations contenues dans les images issues de ladite caméra **endobuccale** de prise d'empreinte optique (4) et les images issues de ladite caméra de visualisation (7),
- à traiter informatiquement, en les corrélant, les premières images avec des secondes images correspondantes à celles prises par la caméra de visualisation (7),
- et à projeter les images corrélées sur ledit verre optique (6),
- **les secondes images étant substituées ou superposées aux premières images, pour permettre une vision directe augmentée par l'indication des zones mesurées pour permettre à l'utilisateur de contrôler l'avancement de son travail.**

2. Dispositif de visualisation (1) selon la revendication 1, **caractérisé en ce qu'**il comprend un système d'éclairage (11) adapté à permettre une prise d'empreinte optique d'organes de la bouche (8) par la caméra de prise d'empreinte optique (4).

3. Dispositif de visualisation (1) selon la revendication 1 ou la revendication 2, **caractérisé en ce que** la corrélation des premières images correspondantes à celles prises par la caméra de visualisation (7) avec les secondes images correspondantes à celles prises par la caméra de prise d'empreinte optique (4) est réalisée par une superposition des premières images avec les secondes images correspondantes.

4. Dispositif de visualisation (1) selon la revendication 1 ou la revendication 2, **caractérisé en ce que** la corrélation des premières images correspondantes à celles prises par la caméra de visualisation (7) avec les secondes images correspondantes à celles prises par la caméra de prise d'empreinte optique (4) est réalisée par un remplacement des premières images par les secondes images correspondantes.

5. Dispositif de visualisation (1) selon l'une des revendications 1 à 4, **caractérisé en ce que** l'unité centrale (10) est adaptée à recevoir, mémoriser et traiter les images prises par la caméra de visualisation (7) et celles prises par la caméra de prise d'empreinte optique (4).

6. Dispositif de visualisation (1) selon l'une des revendications 1 à 5, **caractérisé en ce que** l'unité centrale (10) est configurée pour corréler progressivement, en fonction de la progression de la prise d'empreinte optique, les premières images avec les secondes images.

7. Dispositif de visualisation (1) selon l'une des revendications 5 ou 6, **caractérisé en ce que** l'unité centrale (10) est configurée pour projeter sur le verre optique (6) des informations supplémentaires relatives au patient (2).

8. Dispositif de visualisation (1) selon la revendication 7, **caractérisé en ce que** les informations supplémentaires relatives au patient (2) comprennent des données à respecter pour la réalisation d'une prothèse dentaire.

9. Dispositif de visualisation (1) selon l'une des revendications 7 ou 8, **caractérisé en ce qu'**il comprend au moins un instrument périphérique relié à l'unité centrale (10) et adapté à capter des informations supplémentaires relatifs au patient (2).

10. Dispositif de visualisation (1) selon la revendication 9, **caractérisé en ce que** l'un des instruments périphérique est configuré pour soit capter l'occlusion statique et les mouvements mandibulaires, soit capter la couleur des dents, soit capter la forme du visage, soit capter les données physiologiques du patient.

11. Dispositif de visualisation (1) selon l'une des revendications 7 à 10, **caractérisé en ce qu'**un instrument périphérique est configuré pour analyser des images endobuccales du patient (2) préalablement prises par d'autres instruments périphériques.

12. Dispositif de visualisation (1) selon l'une des revendications 1 à 11, **caractérisé en ce qu'**il comprend un microphone (15) adapté à capter des ordres de commande provenant de l'utilisateur (3) et à les transmettre à l'unité centrale (10).

13. Dispositif de visualisation (1) selon l'une des revendications 1 à 12, **caractérisé en ce qu'**au moins l'un des deux éléments pris parmi la caméra de prise d'empreinte optique (4) et la paire de lunettes à réalité augmentée (5) comprend au moins l'un des trois instruments de mesure pris parmi un accéléromètre, un gyromètre et un magnétomètre.

## Patentansprüche

1. Vorrichtung (1) zum Betrachten des Inneren eines Mundes (8) eines Patienten (2), wobei die Vorrichtung (1) erstens eine Brille mit einer optischen Linse (6), durch die ein Benutzer (3) der Brille das Innere des Mundes (8) sehen kann, zweitens eine Endobukkalkamera (4), die einen optischen Abdruck von im Mund (8) angeordneten Organen aufnehmen kann, und drittens eine Zentraleinheit (10) umfasst, die geeignet ist, auf die optische Linse (6) erste Bilder zu projizieren, die den von der Kamera (4) für den optischen Abdruck aufgenommenen Bildern entsprechen, **dadurch gekennzeichnet, dass** die Brille eine Augmented-Reality-Brille (5) ist, die eine Betrachtungskamera (7) umfasst, die geeignet ist, als Bild das aufzunehmen, was der Benutzer (3) durch die optische Linse (6) sieht, wobei die Zentraleinheit (10) angepasst ist:
- Informationen zu messen, die in den von der Endobukkalkamera (4) für den optischen Abdruck aufgenommenen Bildern und den Bildern der Betrachtungskamera (7) enthalten sind,
- die ersten Bilder, die mit zweiten Bildern korreliert werden, die den von der Betrachtungskamera (7) aufgenommenen entsprechen, mit dem Computer zu verarbeiten,
- und die korrelierten Bilder auf die optische Linse (6) zu projizieren,
- wobei die zweiten Bilder durch die ersten Bilder ersetzt oder überlagert werden, um eine direkte Sicht zu ermöglichen, die durch die Angabe der gemessenen Bereiche verbessert wird, damit der Benutzer den Fortschritt seiner Arbeit kontrollieren kann.

2. Betrachtungsvorrichtung (1) nach Anspruch 1, **dadurch gekennzeichnet, dass** sie ein Beleuchtungssystem (11) umfasst, das geeignet ist, das Aufnehmen des optischen Abdrucks von Organen im Mund (8) durch die Kamera für den optischen Abdruck (4) zu ermöglichen.

3. Betrachtungsvorrichtung (1) nach Anspruch 1 oder 2, **dadurch gekennzeichnet, dass** die Korrelation der ersten Bilder, die denen entsprechen, die von der Betrachtungskamera (7) aufgenommen wurden, mit den zweiten Bildern, die denen entsprechen, die von der Kamera für den optischen Eindruck (4) aufgenommen wurden, durch Überlagerung der ersten Bilder mit den entsprechenden zweiten Bildern erfolgt.

4. Betrachtungsvorrichtung (1) nach Anspruch 1 oder 2, **dadurch gekennzeichnet, dass** die Korrelation der ersten Bilder, die denen entsprechen, die von der Betrachtungskamera (7) aufgenommen wurden, mit den zweiten Bildern, die denen entsprechen, die von der Kamera für den optischen Eindruck (4) aufgenommen wurden, dadurch erreicht wird, dass die ersten Bilder durch die entsprechenden zweiten Bilder ersetzt werden.

5. Betrachtungsvorrichtung (1) nach einem der Ansprüche 1 bis 4, **dadurch gekennzeichnet, dass** die Zentraleinheit (10) geeignet ist, die von der Betrachtungskamera (7) aufgenommenen Bilder und die von der Kamera für den optischen Abdruck (4) aufgenommenen Bilder zu empfangen, zu speichern und zu verarbeiten.

6. Betrachtungsvorrichtung (1) nach einem der Ansprüche 1 bis 5, **dadurch gekennzeichnet, dass** die Zentraleinheit (10) so konfiguriert ist, dass sie in Abhängigkeit vom Fortschritt des optischen Abdrucks die ersten Bilder progressiv mit den zweiten Bildern korreliert.

7. Betrachtungsvorrichtung (1) nach einem der Ansprüche 5 oder 6, **dadurch gekennzeichnet, dass** die Zentraleinheit (10) so konfiguriert ist, dass sie zusätzliche Informationen bezüglich des Patienten (2) auf die optische Linse (6) projiziert.

8. Betrachtungsvorrichtung (1) nach Anspruch 7, **dadurch gekennzeichnet, dass** die zusätzlichen Patienteninformationen (2) Daten umfassen, die für die Herstellung eines Zahnersatzes zu beachten sind.

9. Betrachtungsvorrichtung (1) nach einem der Ansprüche 7 oder 8, **dadurch gekennzeichnet, dass** sie mindestens ein Peripherieinstrument umfasst, das mit der Zentraleinheit (10) verbunden und dazu geeignet ist, zusätzliche Patienteninformationen (2) zu erfassen.

10. Betrachtungsvorrichtung (1) nach Anspruch 9, **dadurch gekennzeichnet, dass** eines der Peripherieinstrumente so konfiguriert ist, dass es entweder statische Okklusions- und Unterkieferbewegungen erfasst, die Zahnfarbe erfasst, die Gesichtsform erfasst oder physiologische Daten des Patienten erfasst.

11. Betrachtungsvorrichtung (1) nach einem der Ansprüche 7 bis 10, **dadurch gekennzeichnet, dass** ein Peripherieinstrument konfiguriert ist, um endobukkale Bilder des Patienten (2) zu analysieren, die zuvor von anderen Peripherieinstrumenten aufgenommen wurden.

12. Betrachtungsvorrichtung (1) nach einem der Ansprüche 1 bis 11, **dadurch gekennzeichnet, dass** sie ein Mikrophon (15) umfasst, das geeignet ist, Steuerbefehle vom Benutzer (3) zu erfassen und sie an die Zentraleinheit (10) zu übertragen.

13. Betrachtungsvorrichtung (1) nach einem der Ansprüche 1 bis 12, **dadurch gekennzeichnet, dass** mindestens eines der beiden von der Kamera für den optischen Abdruck (4) und der Augmented-Reality-Brille (5) aufgenommenen Elemente mindestens eines der drei von einem Beschleunigungsmesser, einem Gyrometer und einem Magnetometer aufgenommenen Messinstrumente umfasst.

## Claims

1. Device (1) for viewing the interior of the mouth (8) of a patient (2), the device (1) comprising, firstly, a pair of spectacles having optical glass (6) through which a user (3) of the pair of glasses can see the inside of the mouth (8), secondly, an **intraoral** camera (4) suitable for taking an optical impression of organs arranged in the mouth (8), and, thirdly, a central processing unit (10) suitable for projecting first images corresponding to those taken by the optical impression camera (4) onto the optical glass (6), **characterized in that** said pair of glasses is a pair of augmented reality glasses (5) comprising a viewing camera (7) suitable for taking an image of what the user (3) sees through the optical glass (6), said central processing unit (10) being suitable for:
- measuring information contained in the images from said **intraoral** optical impression camera (4) and the images from said viewing camera (7),
- computer processing the first images by correlating them with second images corresponding to those taken by the viewing camera (7),
- and projecting the correlated images onto said optical glass (6),
- **the second images being substituted or superimposed on the first images to allow a direct view which is augmented by indicating the areas that have been evaluated to allow the user to monitor the progress of their work.**

2. Viewing device (1) according to claim 1, **characterized in that** it comprises a lighting system (11) suitable for allowing an optical impression of organs in the mouth (8) to be taken by the optical impression camera (4).

3. Viewing device (1) according to either claim 1 or claim 2, **characterized in that** the first images corresponding to those taken by the viewing camera (7) are correlated with the second images corresponding to those taken by the optical impression camera (4) by superimposing the first images with the corresponding second images.

4. Viewing device (1) according to either claim 1 or claim 2, **characterized in that** the first images corresponding to those taken by the viewing camera (7) are correlated with the second images corresponding to those taken by the optical impression camera (4) by replacing the first images with the corresponding second images.

5. Viewing device (1) according to any of claims 1 to 4, **characterized in that** the central processing unit (10) is suitable for receiving, storing and processing the images taken by the viewing camera (7) and those taken by the optical impression camera (4).

6. Viewing device (1) according to any of claims 1 to 5, **characterized in that** the central processing unit (10) is configured to gradually correlate the first images with the second images depending on the progress of the optical impression being taken.

7. Viewing device (1) according to either claim 5 or claim 6, **characterized in that** the central processing unit (10) is configured to project additional information relating to the patient (2) onto the optical glass (6).

8. Viewing device (1) according to claim 7, **characterized in that** the additional information relating to the patient (2) comprises data to be observed for producing a dental prosthesis.

9. Viewing device (1) according to either claim 7 or claim 8, **characterized in that** it comprises at least one peripheral instrument which is connected to the central processing unit (10) and suitable for capturing additional information relating to the patient (2).

10. Viewing device (1) according to claim 9, **characterized in that** one of the peripheral instruments is configured to either capture the static occlusion and the mandibular movements of the patient, or capture the color of the teeth, or capture the shape of the face, or capture the physiological data of the patient.

11. Viewing device (1) according to any of claims 7 to 10, **characterized in that** a peripheral instrument is configured to analyze intraoral images of the patient (2) previously taken by other peripheral instruments.

12. Viewing device (1) according to any of claims 1 to 11, **characterized in that** it comprises a microphone (15) suitable for capturing control commands from the user (3) and transmitting them to the central processing unit (10).

13. Viewing device (1) according to any of claims 1 to 12, **characterized in that** at least one of the two elements from among the optical impression camera (4) and the pair of augmented reality glasses (5) comprises at least one of three measuring instruments from among an accelerometer, a gyrometer and a magnetometer.
